# EUROPEAN PATENT SPECIFICATION

(11) **EP 2 389 956 B1**
(45) Date of publication and mention of the grant of the patent: **26.11.2014**
(21) Application number: 11166893.5
(22) Date of filing: 15.10.2009
(51) Int. Cl.: A61L 12/08, G02C 13/00, A45C 11/00, A61F 9/00

(54) **Contact lens cases for delivery of ophthalmic agents**
Kontaktlinsenetuis zur Abgabe von ophthalmischen Mitteln
Boîtes à lentilles de contact pour distribution d'agents ophthalmiques

(30) Priority: 23.10.2008 US 256526
(43) Date of publication of application: 30.11.2011
(62) Divisional of application: 09740807.4
(73) Proprietor: Bausch & Lomb Incorporated, Rochester, NY 14604 (US)
(72) Inventor: Mao, Shane, Pittsford, NY 14534 (US); Barr, Joseph T., Dublin, OH 43016 (US); Liu, Xiaojun Michael, Pittsford, NY 14534 (US); Groemminger, Suzanne F., Rochester, NY 14609 (US)
(74) Representative: Vossius & Partner

(56) References cited:
- EP-A1- 0 381 616
- FR-A1- 2 542 463

## Description

### TECHNICAL FIELD

The invention is directed to contact lens cases, and in particular, to the delivery of ophthalmic active agents to the eyes by means of contact lenses.

### BACKGROUND

Current methods of delivering ophthalmic active agents, i.e., both pharmaceutical and non-pharmaceutical agents, to treat ocular disorders and diseases are somewhat inefficient and cumbersome. For example, ninety percent of current ophthalmic active agents are provided in drops or ointments, which typically have low absorption rates. In fact, usually less than seven percent of the applied active agent is absorbed by eye tissue. Due to low absorption rates, drops and ointments must include high dosages of active agent(s), and multiple dosages often must be applied in order for the active agent(s) to be effective. Additionally, side effects, such as heart problems, can result when using eye drops because the active agent(s) in the drops can seep into the nasal cavity and then into the bloodstream and other tissues.

Convenience for the patient, and consequently, patient compliance in administering the ophthalmic agents is also an issue to be considered. For example, a person may need to transport one or more containers (such as bottles or tubes) containing eye drop solution or eye ointment to ensure that appropriate treatments are applied at specific times during a day. Also, the person will likely have to administer the drops every two to four hours because of the low absorption rates and tear wash out. Not only is this an inconvenience, but the person may forget or miss one or two treatments each day.

EP 0 381 616 A1 relates to a contact lens disinfection container structure which comprises an open topped lens container, a piercer means mounted in the lens container and having piercing portions thereon projecting to the vicinity of the open top of the lens container, an open topped solution container attached to the lens container for being foldable over against the lens container with the open top thereof facing the open top of the lens container, a sterilizing or disinfecting solution in the solution container, a piercable cover sealed to the edge of the solution container around the open to thereof and a catalyst in the lens container for decomposing the sterilizing agent in the solution.

It is therefore desirable to provide a more effective and convenient system for delivering ophthalmic active agents to the eye. More specifically, it is desirable to provide a system that delivers ophthalmic agents to the eye via a contact lens.

### SUMMARY

The present invention relates to a contact lens case as defined in claim 1. Preferred embodiments are defined in the dependent claims.

### BRIEF DESCRIPTION OF THE DRAWINGS

FIG. 1 is a perspective view of a contact lens case according to the invention;
FIG 2 is an exploded side cross-sectional view of the contact lens case of FIG. 1;
FIG. 3 is a top view of the contact lens case of FIG. 1;
FIG. 4 is a side cross-sectional view of a lid assembly of the contact lens case of FIG. 1;
FIGS. 5, 6 and 7 are top, side cross-sectional and bottom views, respectively, of a dispenser pack of the lid assembly of FIG. 4;
FIG. 8 is a partially transparent perspective view of a contact lens case according to an embodiment of the invention;
FIG. 9 is a perspective view of a lid assembly for a contact lens case according to another embodiment of the invention;

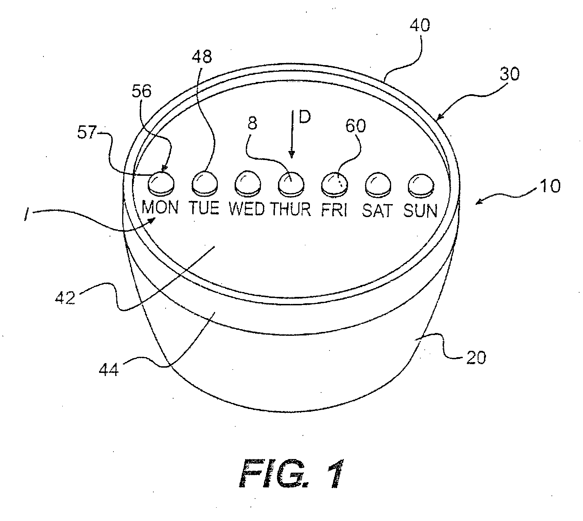

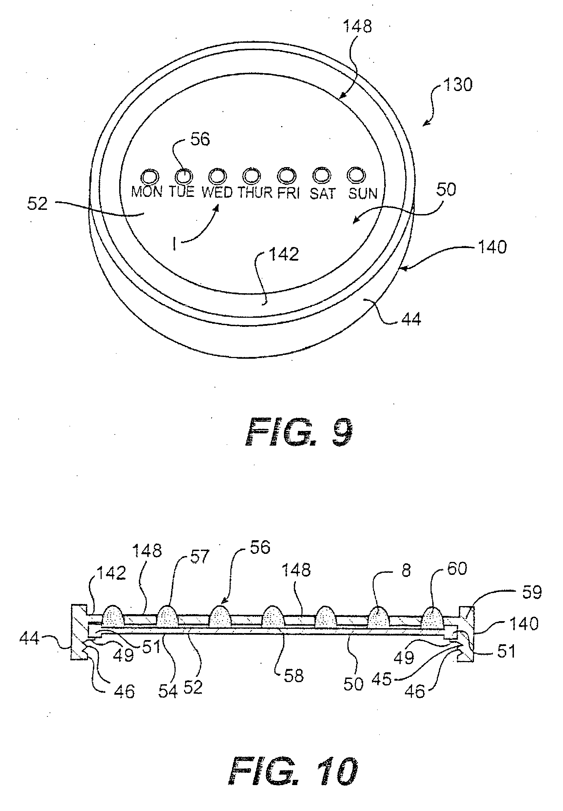

FIG. 1 is a side cross-sectional view of the lid assembly of FIG. 9.

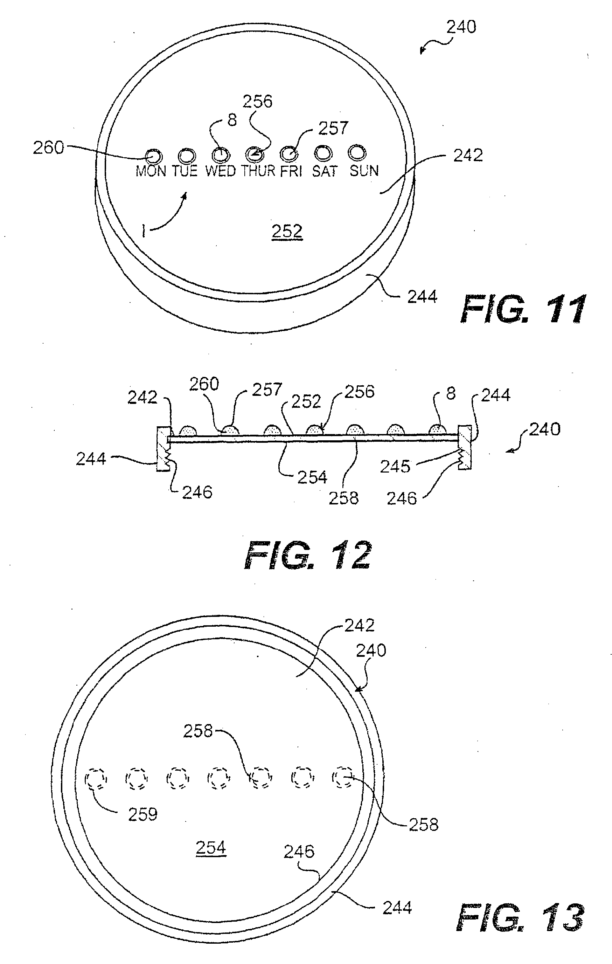

FIG. 11 is a perspective view of a lid for a contact lens case according to another embodiment of the invention;
FIG. 12 is a side cross-sectional view of the lid of FIG. 11;
FIG. 13 shows a bottom side of the lid of FIG. 11;

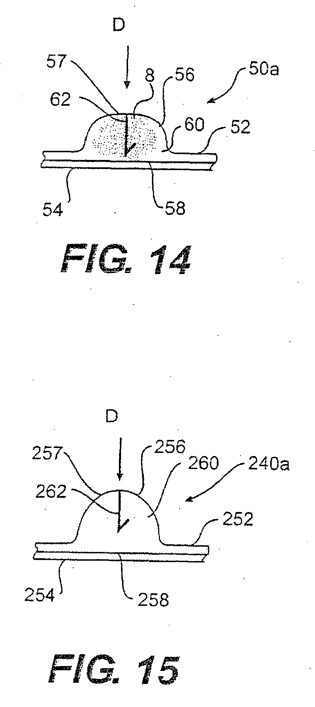

FIG. 14 is an enlarged partial view of a dispenser pack for a lid assembly not according to the invention; and
FIG. 15 is an enlarged partial view of a lid for a contact lens case not according to the invention.

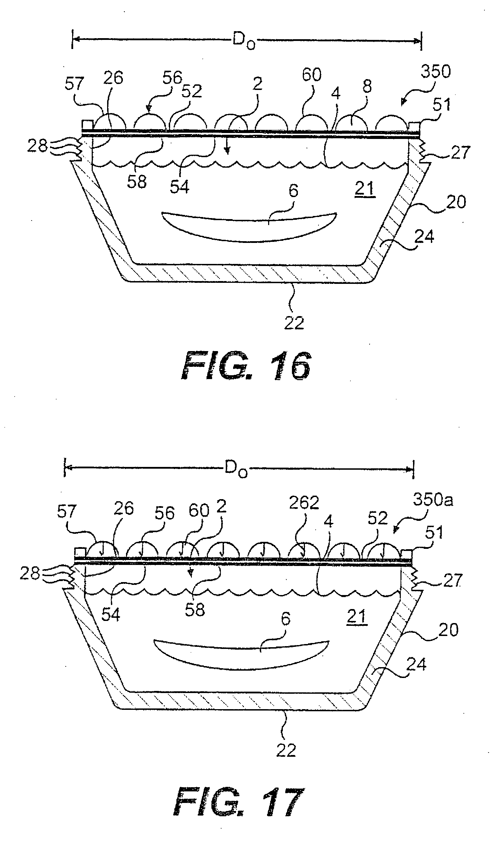

FIG. 16 is a side view showing a dispenser pack disposed on a contact lens case, according to another embodiment of the invention.
FIG. 17 is a side view showing a dispenser pack disposed on a contact lens case, not according to the invention.

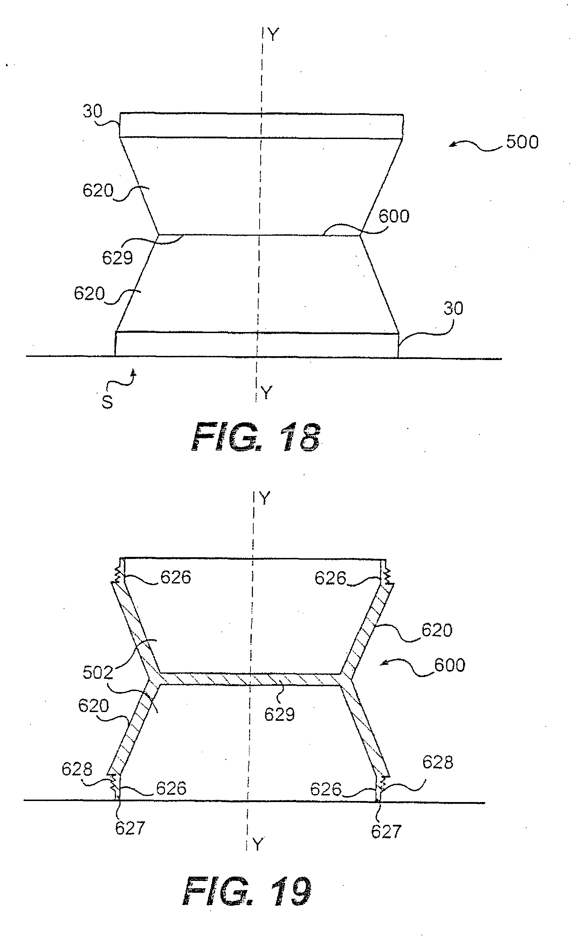

FIG. 18 is a side view of a "vertical" contact lens case according to another embodiment of the invention.
FIG. 19 is a side cross-sectional view of a base of the contact lens case of FIG. 18.

### DETAILED DESCRIPTION

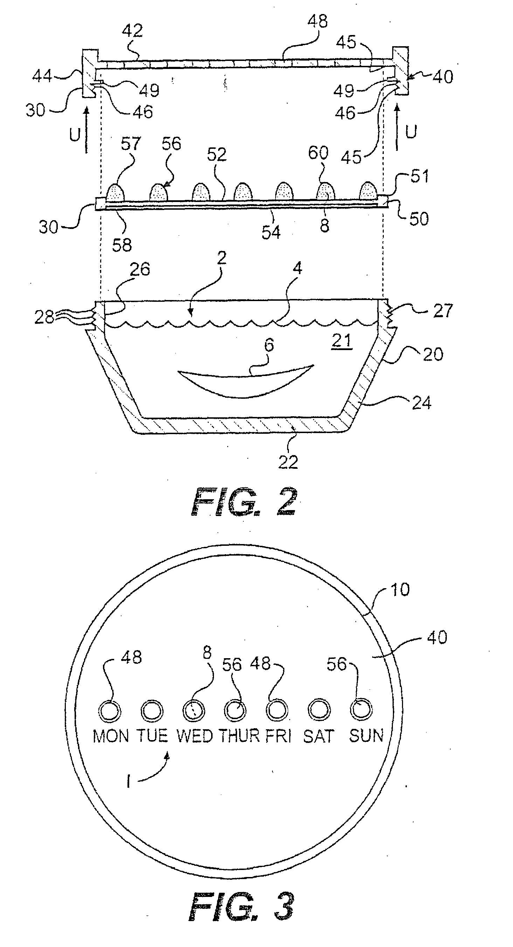

A contact lens case 10 according to the invention is illustrated in FIGS. 1-7. The case 10 includes a container 20 and a removable lid assembly 30 for attachment to the container 20. Referring to FIG. 2, the lid assembly 30 includes a lid 40 and a dispenser pack, or blister pack 50 sized and configured to fit inside the lid 40.

Still referring to FIG. 2, the container 20 may be constructed of a rigid material such as plastic, and includes a bottom wall 22 and a generally annular side wall 24 extending from the bottom wall 22. The bottom wall 22 and side wall 24 define an internal reservoir or cavity 2, which is suitable for holding solution 4 and a contact lens 6 in the solution 4. A lid engaging portion 26 is defined at the top of the side wall 24. A plurality of external threads 28 are provided on an external side 27 of the lid engaging portion 26. The container 20 may be constructed of plastic, however other materials providing suitable weight, durability and rigidity may be used.

Referring to FIGS. 2-4, the lid 40 includes a top wall 42 and an annular side wall 44 extending from the top wall 42. The top wall 42 includes a plurality of openings 48. A plurality of internal threads 46 are provided on an internal surface 45 of the side wall 44, and are designed to engage the external threads 28 to allow the lid 40 to be threaded onto and off of the container 20 to cover and uncover, respectively, the reservoir 2. A retaining ledge 49 extends from the internal surface 45. The retaining ledge 49 may comprise a continuous annular projection or a plurality of spaced projections. The lid 40 may be constructed of plastic or another material that has a suitable weight, durability and rigidity.

According to alternate embodiments of the invention (not shown), the threads 46 on the lid 40 and the threads 28 on the lid engaging portion 26 of the container 20 may be eliminated, and the lid 40 may be configured to fit onto the lid-engaging portion 26 by being pressed or snapped onto the lid engaging portion 26 with an interference or friction fit.

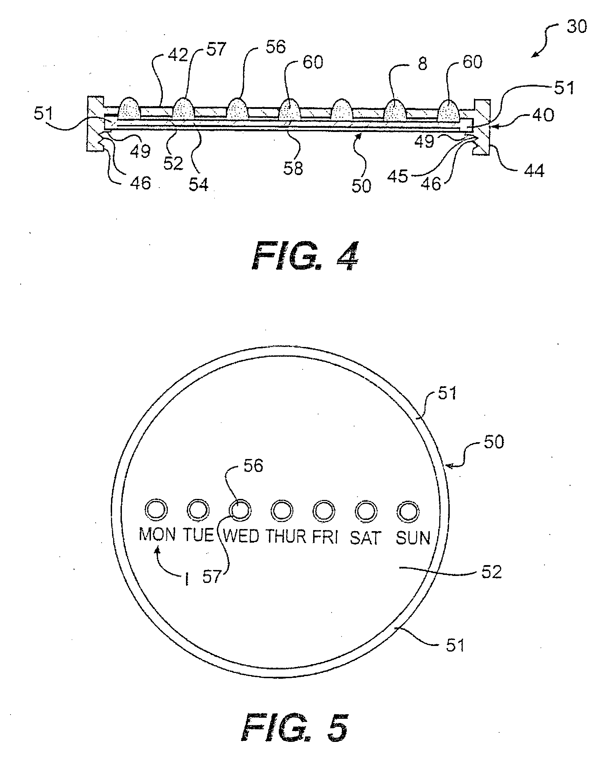

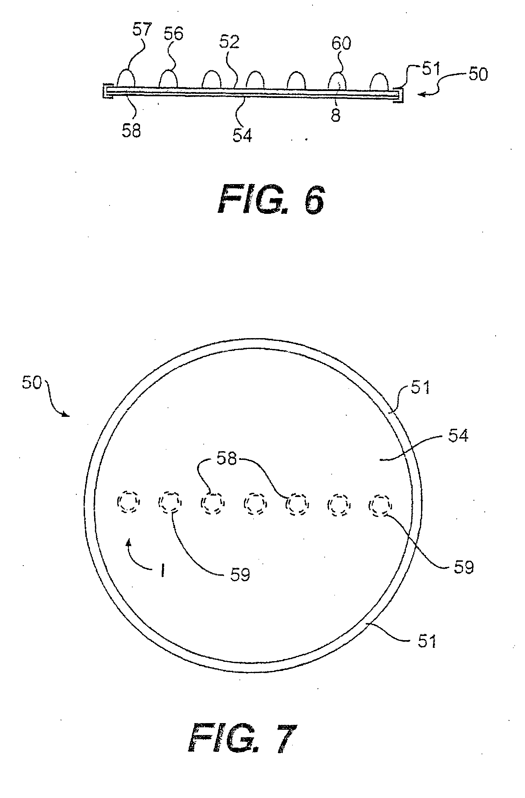

As shown in FIGS. 5-7, the dispenser pack 50 is a substantially disc-shaped member formed by an upper sheet of material 52 attached to a lower sheet of material 54. The pack 50 includes a plurality of bubble-shaped compartments, or blisters 56 are defined by the upper sheet of material 52 and the lower sheet of material 54. More specifically, the upper sheet of material 52 defines the top walls 57 of the compartments 56, while the lower sheet of material 54 defines the bottom walls 58 (FIG. 7) of the compartments 56. The top walls 57 may be convex and the bottom walls 58 may be substantially flat, thereby defining an interior volume 60 of each compartment 56. The bottom walls 58 may include patterns of weakness 59. The patterns of weakness 59 may comprise nicks, scores, cuts, perforations or combinations thereof. In the embodiment shown, the patterns of weakness 59 are circular in shape, however patterns of different shapes and sizes may be provided. The upper sheet of material 52 may be constructed of a deformable material such as plastic, for example, and preferably a translucent or transparent plastic. The lower sheet of material 54 may be rupturable, and may be constructed of a material that is impermeable to water, such as foil or plastic.

Each compartment 56 contains a treatment unit 8 contained in its interior volume 60. The patterns of weakness 59 facilitate rupturing of the bottom walls 58 to dispense the treatment units 8 from the compartments 56.

The pack 50 may further include an annular gasket 51 positioned at the outer periphery of the pack 50 for providing a seal between the lid 40 and the container 20. The gasket 51 may cover portions of the upper and lower sheets of material 52, 54 and/or the outer peripheral edge of the pack 50. The gasket 51 may be constructed of a resilient material such as rubber, silicone, or plastic, for example.

Referring to FIGS. 2 and 4, the lid assembly 30 is assembled by inserting the pack 50 through the bottom of the lid 40 (in direction U, shown in FIG. 2) inside the side wall 44, securing the peripheral edge of the pack 50 above the retaining ledge 49, aligning each compartment 56 with a respective opening 48 in the top wall 42, and fitting the top walls 57 of the compartments 56 through the openings. If the gasket 51 is provided on the pack 50, the gasket 51 forms a tight seal between the pack 50 and the lid 40. When the pack 50 is inserted into the lid 40, the top walls 57 of the compartments 56 protrude through the openings 48 and are at least partially exposed at the top of the lid 40, and the bottom walls 58 are exposed at a bottom side of the lid 40.

Once the lid assembly 30 is assembled, the lid assembly 30 may be threaded or pressed (where the lid 40 and container 20 are threadless) onto the container 20 after the solution 4 and the contact lens 6 are placed in the reservoir 2 in order to close the case 10 and prepare the case 10 for delivering a quantity of a treatment agent from a treatment unit 8 to the contact lens 6. A treatment unit 8 may be individually dispensed from a compartment 56 by pressing the top wall 57 of the compartment 56 downward (in direction D, shown in FIG. 1) such that the top wall 57 and the unit 8 move downward to thereby rupture the bottom wall 58, and release the unit 8 into the reservoir 2. After the unit 8 is released into the reservoir 2, the lens 6 is allowed to soak in the solution 4 for a period sufficient to allow the unit 8 to dissolve into or mix with the solution 4, and to allow at least some of the solution 4 and at least some of the agent from the unit 8 to be absorbed or retained by the contact lens 6. The time required for soaking will vary depending on the composition of the solution 4, the lens 6, and the form and composition of the unit 8. After soaking, the contact lens 6 may be placed in a users eye for treating the eye with the agent.

It should be noted that the treatment unit 8 may also be dispensed from a compartment 56 prior to securing the lid assembly 30 to the container 20, while the lid 40 is detached and from the container 20 and held thereabove.

Treatment units 8 may be administered to the contact lens 6 in the above-described manner at daily intervals, or at any other prescribed intervals, depending on the form and composition of the unit 8 and the condition being treated. In order to remind a user when a treatment is due, the lid 40 and/or dispenser pack 50 may include treatment identifiers or labels I (FIGS. 1, 3 and 5) on its outer surface, in the form of words, abbreviated words (such as Mon, Tue, Wed, Thur, Fri, Sat, Sun), numbers or symbols indicative of treatment days, treatment numbers or treatment times. Subsequent treatments are applied to a lens 6 by dispensing units 8 from unused compartments 56 at the prescribed time intervals. Where the upper layer 52 of the pack 50 is translucent or transparent, it is easy for a user to determine visually whether a compartment 56 is empty or whether a compartment 56 contains any undispensed units 8. In the embodiment shown, the pack 50 includes seven compartments, which is suitable for applying daily treatments over the course of one week. Once the units 8 have been dispensed from all of the compartments 56, a new pack 50 may be inserted into the lid 40.

The described contact lens cases can also be one component of a drug delivery kit. For example, a kit would include a case 10 and a plurality of packs 50 such as four packs containing seven compartments 56. The four packs would provide one month of eye treatments before the case 10 is discarded and replaced. Proper hygiene is promoted by limiting the number of packs 50, and therefore the number of eye treatments, provided with the case 10. It should be understood, however, that any number of packs 50 may be provided, and each pack 50 may be provided with any number of compartments 56, as desired.

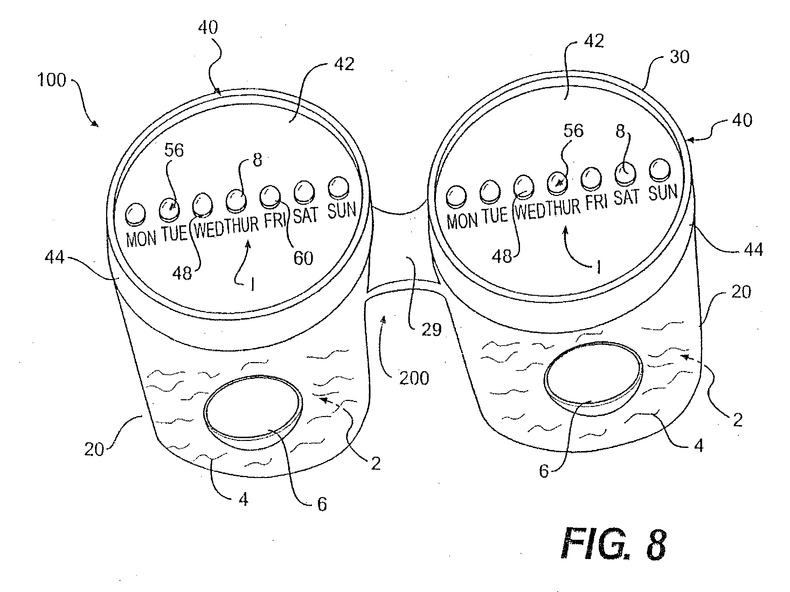

FIG. 8 shows a contact lens case 100 according to an embodiment of the invention. In FIG. 8, reference characters repeated from the description of FIGS. 1-7 indicate similar features to which the description of FIGS. 1-7 applies. The case 100 includes a base 200 having two containers 20 connected to each other by a center span 29. Each container 20 may contain solution 4 and a contact lens 6 in a reservoir 2. A pair of lid assemblies 30 are provided for attachment to the containers 20 and dispensing treatment units 8 from compartments 56 in the same manner discussed in the embodiment of FIGS. 1-7. According to this embodiment, in order to allow for approximately one-month of daily eye treatments, the case 100 may be provided with eight packs 50 (four for each eye/lid assembly 30), each containing seven compartments 56. As in the previous embodiment, any number of packs 50 may be provided, and the packs 50 may include any number of compartments 56.

FIGS. 9 and 10 show a lid assembly 130 according to another embodiment of the invention, wherein reference numbers repeated from the lid assembly 30 of the previous embodiment indicate similar features. The lid assembly 130 includes a lid 140 and a dispenser pack 50. The lid 140 is similar to the lid 40 of the embodiments of FIGS. 1-8, except that the lid 140 has a top wall 132 with a single central opening 148 through which all of the compartments 56 are exposed on a top side of the lid 40. The lid assembly 30 is assembled in a manner similar to the way in which the previously described lid assembly 30 is assembled, except that the top walls 57 of all of the compartments 56 are inserted through the opening 148.

FIGS. 11-13 show a lid 240 according to another embodiment of the invention. The lid 240 may be used in place of the lid assemblies 30, 130 of the previously described embodiments. The lid 240 includes a top wall 242 and an annular side wall 244 extending from the top wall 242. The top wall 242 may be formed from an upper sheet of material 252 and a lower sheet of material 254 attached to the upper sheet of material 252 (FIG. 12). The upper sheet of material 252 may be constructed of a plastic, preferably translucent or transparent plastic, while the lower sheet of material 254 may be constructed of foil or plastic. The upper and lower sheets of material 252, 254 may be bonded to the side wall 244 such that the lid 240 is formed as a one-piece body.

A plurality of internal threads 246 are provided on an internal surface 245 of the side wall 244, and are designed to engage the external threads 28 of a container 20 as described the in the embodiments of FIGS. 1-8 in order to allow the lid 240 to engage or disengage the container 20 to cover and uncover, respectively, the reservoir 2 by turning the lid 240. As is the case with the lids 40, 140 the threads 246 may be eliminated from the lid 240 in order to provide a press-on, interference fit between the lid 240 a container 20 that lacks threads 28.

The top wall 242 includes a plurality of compartments 256 defined by the upper and lower sheets of material 252, 254. Each of the compartments 256 may contain a treatment unit 8 that can be dispensed from the compartment 256. The upper sheet of material 252 defines the top walls 257 of the compartments 256, and the lower sheet of material 254 defines the bottom walls 258 (see FIGS. 12 and 13) of the compartments 256. The top walls 257 may be convex and the bottom walls 258 may be substantially flat, thereby defining an interior volume 260. The bottom walls 58 may include patterns of weakness 259, which may comprise nicks scores, cuts, perforations or combinations thereof. Although the patterns of weakness 259 are shown circular in shape, patterns of different shapes and sizes may be provided. The upper sheet of material 252 may be constructed of a deformable material such as plastic, for example, and preferably a translucent or transparent plastic. The lower sheet of material 254 may be rupturable, and may be constructed of a material that is impermeable to water, such as foil or plastic.

The lid 240 is a one-piece alternative to the lid assemblies 30, 130 described above with respect to FIGS. 1-10. Therefore, the entire lid 240 must be replaced when the treatment units 8 have been dispensed from all of the compartments 256 in the lid 240. As in the embodiments of FIGS. 1-8, a kit containing a one-month supply of treatments for a single eye may include four lids 240, wherein each lid 240 contains seven compartments 256. A kit, for example, containing a one-month supply of treatments for two eyes may include eight lids 240, wherein each lid 240 contains seven compartments 256. However, any number of lids 240 having any number of compartments 256 may be provided, as desired.

A unit 8 can be dispensed from a compartment 256 in the same way a unit 8 is dispensed from a compartment 56 in the previous embodiments. Specifically, pressing down on the top wall 257 in the direction D will force the top wall 257 and the unit 8 downward and thereby rupture the bottom wall 258 of the compartment 256. To help ensure proper administration of treatments, the lid 240 may also include treatment identifiers or labels I (FIG. 11) on its outer surface corresponding to each compartment 256.

FIGS. 14 and 15 respectively show a dispenser pack 50a and a lid 240a according to embodiments which are not within the scope of the present invention. The pack 50a and lid 240a are similar to the previously described pack 50 and lid 240, respectively, except that the compartments 56, 256 include puncturing elements 62, 262. The puncturing elements 62, 262 are attached to interior surfaces of the top walls 57, 257, and extend downward into the interior volumes 60, 260. The puncturing elements 62, 262 may each comprise a rod, pin, blade, or any other projection that is sufficiently sharp to puncture the bottom walls 58, 258. According to the embodiments of FIGS. 14 and 15, the puncturing elements 62, 262 facilitate the dispensing of a treatment unit 8 from the compartments 56, 256 by moving downward into contact with the bottom walls 258 when the top walls 257 are depressed, and then puncturing the bottom walls 258.

FIG. 16 illustrates a dispenser pack 350 according to another embodiment of the invention, wherein reference numbers repeated from the pack 50 (FIGS. 2 and 4) indicate similar features. The dispenser pack 350 is similar to the previously described dispenser pack 50, except that the outer diameter Dₒ of the dispenser pack 350 is selected such that the pack 350 rests on top of the lid engaging portion 26 of a container 20 (FIG. 2) and is sandwiched between the container 20 and a lid 40/140 (FIGS. 2 and 4/FIGS. 9 and 10) when the lid 40/140 is secured onto the container 20.

FIG. 17 illustrates a dispenser pack 350a for use with the lids 40, 140, according to yet another embodiment which is not in the scope of the present invention. The dispenser pack 350a is similar to the dispenser pack 50a (FIG. 14), as indicated by shared reference characters, except that the outer diameter Dₒ of the dispenser pack 350a is selected such that the pack 350a rests on top of the lid engaging portion 26 of a container 20 (FIG. 2) and is sandwiched between the container 20 and a lid 40/140 when the lid 40/140 is secured onto the container 20.

FIGS. 18 and 13 illustrate a contact lens case 500 according to yet another embodiment of the invention. The case 500 includes a base 600 having two containers 620 joined together by a central partition wall 629. Each container 620 includes an annular side wall 624 extending from the partition wall 629, and which combines with the partition wall 629 to define an internal reservoir or cavity 602 for holding solution 4 and a contact lens 6 in the solution 4. As shown in FIG. 19, a lid engaging portion 626 is defined at the top of the side wall 624. A plurality of external threads 628 are provided on an external side 627 of the lid engaging portion 626. The base 600 may be constructed of plastic, however other materials providing suitable weight, durability and rigidity may be used.

A lid assembly 30 may be attached to each container 620 in the same manner described with respect to the container 20 in the embodiment of FIGS. 1-7. The case 500 may be referred to as a "vertical lens case," because the case is configured to rest on a surface S in a storage position, with either of the lids 40 supporting the case 500 on the surface S, and the containers 620 aligned with each other along a vertical axis Y. Thus, the containers 620 are vertically stacked when the case 500 is in a storage position, and the reservoirs 502 are disposed on vertically opposite sides of the partition wall 629. It is noted that the base 600 is not limited to use with the lid assemblies 30 described in the embodiment of FIGS. 18 and 19, but may also be used with any of the lid assemblies, lids and dispenser packs described in the other various embodiments herein.

The treatment units 8 to be administered to contact lenses 6 according to the various embodiments disclosed herein can be in powder, tablet, liquid or liquid emulsion form, and can contain any ophthalmic agent or compound that is used to treat any ocular disease or any ocular condition. Accordingly, the agent(s) in the units 8 can be selected from any class of compounds, for example, anti-inflammatory agents, anti-infective agents (including antibacterial, antifungal, antiviral, antiprotozoal agents), anti-allergic agents, antiproliferative agents, anti-angiogenic agents, anti-oxidants, neuroprotective agents, cell receptor agonists, cell receptor antagonists, immunomodulating agents, immunosuppressive agents, IOP lowering agents (anti-glaucoma), beta adrenoceptor antagonists, alpha-2 adrenoceptor agonists, carbonic anhydrase inhibitors, cholinergic agonists, prostaglandins and prostaglandin receptor agonists, AMPA receptor antagonists, NMDA antagonists, angiotensin receptor antagonists, somatostatin agonists, mast cell degranulation inhibitors, alpha-2 adrenoceptor antagonists, thromboxane A2 mimetics, protein kinase inhibitors, prostaglandin F derivatives, prostaglandin-2 alpha antagonists and muscarinic agents. It should be understood that while each of the units 8 may comprise the same active agent(s), it is possible to provide different active agents in individual units 8, as desired.

Of particular interest are pharmaceutical active agents that are known to treat an ocular disease or disorder including, but not limited to, a posterior-segment disease or disorder. In certain embodiments, such disease or disorder typically may include diabetic retinopathy, diabetic macular edema, cystoid macular edema, age macular degeneration (including the wet and dry form), optic neuritis, retinitis, chorioretinitis, intermediate and posterior uveitis and choroidal neovascuralization.

Glaucoma is a group of diseases that are characterized by the death of retinal ganglion cells ("RGCs"), specific visual field loss, and optic nerve atrophy. Glaucoma is the third leading cause of blindness worldwide. An intraocular pressure ("IOP") that is high compared to the population mean is a risk factor for the development of glaucoma. However, many individuals with high IOP do not have glaucomatous loss of vision. Conversely, there are glaucoma patients with normal IOP. Therefore, continued efforts have been devoted to elucidate the pathogenic mechanisms of glaucomatous optic nerve degeneration.

It has been postulated that optic nerve fibers are compressed by high IOP, leading to an effective physiological axotomy and problems with axonal transport. High IOP also results in compression of blood vessels supplying the optic nerve heads ("ONHs"), leading to the progressive death of RGCs. See; e.g., M. Rudzinski and H.U. Saragovi, Curr. Med. Chem.―Central Nervous System Agents, Vol. 5, 43 (2005).

Pharmaceutical active agents that are prescribed by a physician for the treatment of glaucoma, and that may be formulated and disposed in the compartmentalized lens case for delivery to a contact lens and subsequently to the eye of a patient include travoprost, brimonidine, levobunolol, epinephrine, bitmatoprost, dipivefrin, carteolol and metipranolol.

In one embodiment, the anti-glaucoma pharmaceutical agent is of general formula II 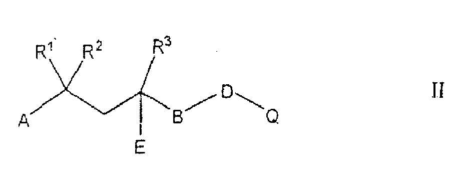 wherein A and Q are independently selected from the group consisting of aryl and heteroaryl groups substituted with at least a halogen atom, cyano group, hydroxy group, or C₁-C₁₀ alkoxy group; R¹, R², and R³ are independently selected from the group consisting of unsubstituted and substituted C₁-C₅ alkyl groups; B is a C₁-C₅ alkylene group; D is the -NH or -NR'- group, wherein R' is a C₁-C₅ alkyl group; and E is the hydroxy group.

Exemplary, pharmaceutical agents of general formula II include A as a dihydrobenzofuranyl group substituted with a fluorine atom; Q as a quinolinyl or isoquinolinyl group substituted with a methyl group; R¹ and R² are independently selected from the group consisting of unsubstituted and substituted C₁-C₅ alkyl groups; B is a C₁-C₃ alkylene group; D is the -NH- group; E is a hydroxy group; and R³ is a trifluoromethyl group.

Exemplary compounds include a glucocorticoid receptor agonist having Formulae III or IV, as disclosed in US Patent Application Publication 2006/0116396. 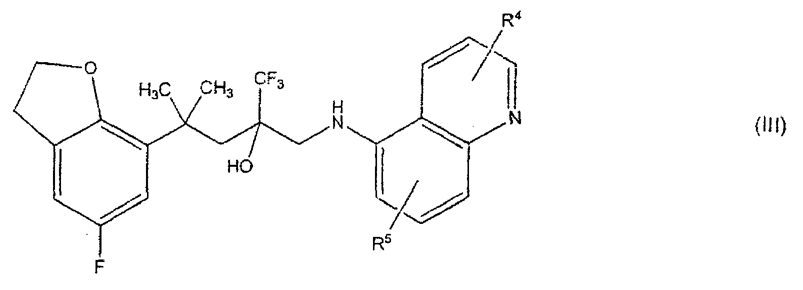 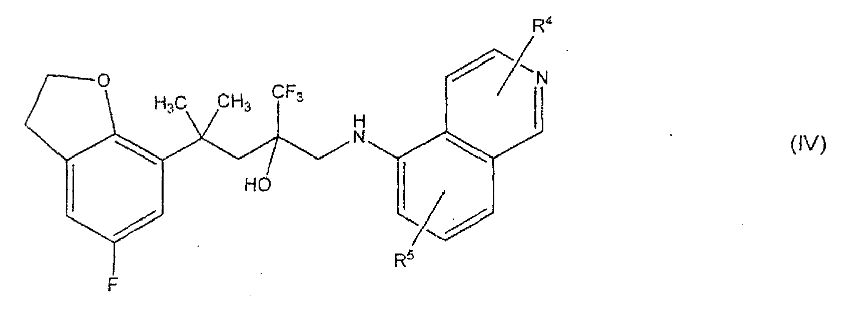 wherein R⁴ and R⁵ are independently selected from the group consisting of hydrogen, halogen, cyano, hydroxy, C₁-C₁₀ (alternatively, C₁-C₅ or C₁-C₃) alkoxy groups, unsubstituted C₁-C₁₀ (alternatively, C₁-C₅ or C₁-C₃) linear or branched alkyl groups, substituted C₁-C₁₀ (alternatively, C₁-C₅ or C₁-C₃) linear or branched alkyl groups, unsubstituted C₃-C₁₀ (alternatively, C₃-C₆ or C₃-C₅) cyclic alkyl groups, and substituted C₃-C₁₀ (alternatively, C₁-C₆ or C₃-C₅) cyclic alkyl groups.

Compositions of the invention also include ocular formulations prescribed by or recommended by a physician, or a health care provider, to treat ocular allergic conditions. Allergy is characterized by a local or systemic inflammatory response to allergens. Allergic conjunctivitis is a disorder that is characterized by the clinical signs and symptoms of eye itching, redness, tearing, and swelling. An estimated 20% of the population in the United States suffer from inflammation of the eye. The signs and symptoms of allergic conjunctivitis can significantly impact the quality of life of patients, from social interactions, productivity at work and school, to the ability to perform visual tasks such as working on a computer or reading.

Currently, available pharmaceutical treatments for inflammation of the eye or symptoms of inflammation of the eye include (1) antihistamines, (2) drugs that block the release of histamine and other substances from a mast cell (e.g., mast cell stabilizers), (3) drugs with multiple modes of action (e.g. antihistamine/mast cell stabilizing agents), and (4) drugs that can actively constrict blood vessels thus reducing redness and swelling (e.g., vasoconstrictors). Additionally, artificial tears have been used to wash the eye of allergens.

The desirability of a particular treatment for inflammation of the eye can be measured against the following factors (1) efficacy at onset of action, (2) duration of action, (3) efficacy at controlling signs and symptoms of allergic conjunctivitis, and (4) comfort of the drop when instilled in the eye.

Pharmaceutical active agents that are prescribed by a physician for the treatment of an ocular allergic condition, and that may be formulated and disposed in the compartmentalized lens case for delivery to a contact lens and subsequently to the eye of a patient include olopatadine, nedacromil, and lotepdrenol.

In one embodiment, the pharmaceutical active agent is ketotifen or a salt thereof. Ketotifen or any ophthalmically acceptable ketotifen salt may be used in the compartmentalized lens case herein described, although ketotifen fumarate is preferred. Ketotifen fumarate is represented by the following formula:

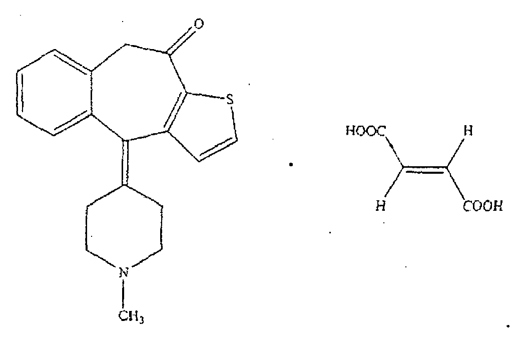

In another embodiment, the pharmaceutical active agent is an anti-redness agent, which may relieve redness in the eye. The preferred anti-redness agent is naphazoline or an ophthalmically acceptable salt thereof such as, for example, naphazoline hydrochloride. Other anti-redness agents that may be used include, but are not limited to, tetrahydrozoline, ephedrine, phenylephrine, oxymetazoline, xylometazoline, pseudoephedrine, tramazoline, other vasoconstrictors, combinations thereof, as well as ophthalmically acceptable salts thereof (e.g., tetrahydrozoline hydrochloride).

Naphazoline hydrochloride is represented by the following formula:

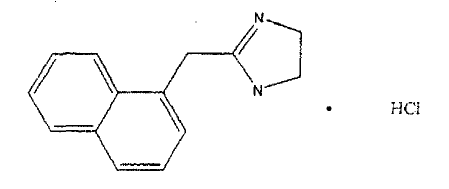

Naphazoline or a naphazoline salt may be present in a concentration from about 0.001% to about 0.2% (or alternatively, from about 0.001% to about 0.1%). In one embodiment, naphazoline or a naphazoline salt is present in a composition at a concentration from about 0.01% to about 0.1%; preferably, from about 0.01% to about 0.07%; more preferably, from about 0.02% to about 0.06%. In some embodiments, the method provides stability to compositions comprising naphazoline or a naphazoline salt in a concentration such that the concentration of naphazoline in the composition is about 0.02% to about 0.05%. Concentrations of a naphazoline salt yielding such concentrations of naphazoline base may be readily calculated; for example, using naphazoline hydrochloride in a concentration of about 0.025% in the composition provides a concentration of naphazoline base in the composition of 0.021%.

Additional information on formulations containing ketotifen, naphazoline or a corresponding pharmaceutically salt of each thereof can be found in U.S. patent application serial no. 10/972,571 filed October 25, 2004.

Pharmaceutical active agents that are prescribed by a physician for the treatment of an ocular infection, and that may be formulated and disposed in the compartmentalized lens case for delivery to a contact lens and subsequently to the eye of a patient include antimicrobial agents, antibiotic agents and antifungal agents. The antimicrobial agents are selected from the group consisting of ciprofloxacin, sulfacetamide, trimethoprin, polymyxin B and norfloxacin. The antibiotic agents are selected from the group consisting of natamycin, tobramycin, gentamicin, gatifloxscin and ofloxacin. One of the more preferred antfungal agents is cromolyn.

Pharmaceutical active agents that are prescribed by a physician for the treatment of ocular inflammation, and which are formulated and disposed in the compartmentalized lens case for delivery to a contact lens and subsequently to the eye of a patient include stearoidal anti-inflammatory agents including dexamethasone, prednisolone, fluormetholone, medrysone, flurbiprofen and loteprednol. Alternatively, a non-steroidal anti-inflammatory agent such as ketorolac can be used with the compartmentalized lens case.

In yet another embodiment, cyclosporine can be formulated into stable emulsions and disposed in the compartmental lens case herein described. Cyclosporine is an immunosuppressive agent that is prescribed to patients with an ocular infection associated with keraconjunctivitis sicca. The cyclosporine is believed to act as a partial immunomodulator and enhances tear production.

In yet another embodiment, pharmaceutical active agents of the FK506 class can be formulated into stable emulsions and disposed in the compartmental lens case herein described. Emulsions, since they contain an aqueous phase, are much less occlusive than oil-based compositions and hence are better tolerated in many situations. Accordingly, in one embodiment a formulation, in the form of an emulsion, comprises a compound of the FK506 class, and a physiologically acceptable alkanediol, ether diol or diether alcohol containing up to 8 carbon atoms as solvent. A compound of the "FK506 class" is a compound which has the basic structure as FK506 and which has at least one of the biological properties of FK506 (e.g., immunosuppressant properties). The compound may be in free base form or pharmaceutically acceptable, acid addition, salt form. A preferred compound of the FK 506 class is disclosed in EP 427 680, e.g. Example 66a (also called 33-epi-chloro-33-desoxyascomycin).

In other embodiments, the agent(s) in treatment units 8 may include non-pharmaceutical ocular agents. For example, the units 8 may comprise agents such as lens rewetting agents, lubricating agents, moisturizing agents, alginate, HA [?], comfort agents, etc. The units 8 may also include disinfectant powders or tablets with rapid dissolution.

## Claims

1. A contact lens care comprising:
a first container (20) defining a first reservoir (2);
a first lid assembly (30) having a first lid (40) attachable to the first container (20) for closing the first reservoir (2), and a first dispenser pack (50) with at least one first compartment (56) that is attachable to the first lid (40) or the first container (20), the first lid assembly (30) is configured such that a top wall (57) of the at least one first compartment (56) is at least partially exposed at a top side (42) of the first lid (40) following attachment of the first dispenser pack (50);
wherein the at least one first compartment (56) comprises an upper sheet of material (52) defining a deformable top wall (57), and a lower sheet of material (54) attached to the upper sheet of material (52) defining a rupturable bottom wall (58), and the bottom wall includes patterns of weakness (59) selected from nicks, scores, cuts, perforations or combinations thereof, configured to rupture upon the application of downward pressure to the top wall (57) of the at least one first compartment (56), and the at least one first compartment (56) contains a first treatment unit (8) for dispensing into the first reservoir (2) at least one compound that is used to treat an ocular disease or ocular condition.

2. The contact lens case of claim 1, wherein the first upper sheet of material (52) is constructed of transparent or translucent plastic, and wherein the first lower sheet of material (54) is constructed of foil or plastic.

3. The contact lens case of claim 1 or 2 comprising:
a second container (20) defining a second reservoir (2);
a second lid assembly (30) having a second lid (40) attachable to the second container (20) for closing the second reservoir (2), and a second dispenser pack (50) with at least one second compartment (56) that is attachable to the second lid (40) or the second container (20), the second lid assembly (30) is configured such that a top wall (57) of the at least one second compartment (56) is at least partially exposed at a top side (42) of the second lid (40) following attachment of the second dispenser pack (50);
wherein the at least one second compartment (56) comprises an upper sheet of material (52) defining a deformable top wall (57), and a lower sheet of material (54) attached to the upper sheet of material (52) defining a rupturable bottom wall (58), and the bottom wall includes patterns of weakness (59) selected from nicks, scores, cuts, perforations or combinations thereof, configured to travel downward to rupture the bottom wall of the at least one second compartment upon the application of downward pressure to the top wall (57) of the at least one second compartment (56), and the at least one second compartment (56) contains a second treatment unit (8) for dispensing into the second reservoir (2) at least one compound that is used to treat an ocular disease or ocular condition.

4. The contact lens case of claim 3, wherein the at least one first compartment and the at least second compartment each comprise seven compartments for a weekly dosage schedule to each eye.

5. The contact lens case of any of claims 1 to 4, wherein the top wall (57) of the at least one first compartment (56) is convex.

6. The contact lens case of any of claims 1 to 5, wherein the first treatment unit (8) comprises a compound used to treat an ocular disease or ocular condition selected from the group consisting of an ocular infection, ocular inflammation, posterior segment disease and glaucoma.

7. The contact lens case of any of claims 1 to 6, wherein the first treatment unit (8) comprises a non-pharmaceutical ocular agent selected from the group consisting of lubricating agents moisturing agents and comfort agents, preferably, alginate or hyaluronic acid.

8. A kit comprising:
the contact lens case of any of claims 1 to 7 having any number of dispenser packs.

9. The kit of claim 8, comprising a packaged contact lens solution to be added to the first reservoir and the second reservoir.

10. The kit of any of claims 8 or 9, wherein the ocular disease or ocular condition is selected from the group consisting of an ocular infection, ocular inflammation, posterior segment disease and glaucoma.

## Patentansprüche

1. Kontaktlinsenetui mit:
einem ersten Behälter (20), der ein erstes Reservoir (2) begrenzt;
einer ersten Deckelanordnung (30) mit einem an dem ersten Behälter (20) anbringbaren ersten Deckel (40) zum Schließen des ersten Reservoirs (2) und mit einer an dem ersten Deckel (40) oder an dem ersten Behälter (20) anbringbaren ersten Spenderpackung (50), die mindestens eine erste Kammer (56) aufweist, wobei die erste Deckelanordnung (30) derart konfiguriert ist, dass an einer oberen Seite (42) des ersten Deckels (40) eine obere Wand (57) der mindestens einen ersten Kammer (56) mindestens teilweise unbedeckt ist, nachdem die erste Spenderpackung (50) angebracht ist;
wobei die mindestens eine erste Kammer (56) eine obere Materialschicht (52), die eine verformbare obere Wand (57) bildet, und eine an der oberen Materialschicht (52) angebrachte untere Materialschicht (54), die eine brechbare untere Wand (58) bildet, aufweist, und wobei die untere Wand Schwächemuster (59) wie Kerben, Rillen, Einschnitte, Perforationen oder eine Kombination davon aufweist, die brechen sollen, wenn Druck nach unten auf die obere Wand (57) der mindestens einen ersten Kammer (56) angewendet wird, und wobei die mindestens eine erste Kammer (56) eine erste Behandlungseinheit (8) enthält, um mindestens eine Substanz, die zur Behandlung einer Augenerkrankung oder eines Augenzustands verwendet wird, in das erste Reservoir (2) abzugeben.

2. Kontaktlinsenetui nach Anspruch 1, wobei die erste obere Materialschicht (52) aus einem durchsichtigen oder halbdurchsichtigen Kunststoff hergestellt ist und wobei die erste untere Materialschicht (54) aus Folie oder Kunststoff hergestellt ist.

3. Kontaktlinsenetui nach Anspruch 1 oder 2 mit:
einem zweiten Behälter (20), der ein zweites Reservoir (2) begrenzt;
einer zweiten Deckelanordnung (30) mit einem an dem zweiten Behälter (20) anbringbaren zweiten Deckel (40) zum Schließen des zweiten Reservoirs (2) und mit einer an dem zweiten Deckel (40) oder an dem zweiten Behälter (20) anbringbaren zweiten Spenderpackung (50), die mindestens eine zweite Kammer (56) aufweist, wobei die zweite Deckelanordnung (30) derart konfiguriert ist, dass an einer oberen Seite (42) des zweiten Deckels (40) eine obere Wand (57) der mindestens einen zweiten Kammer (56) mindestens teilweise unbedeckt ist, nachdem die zweite Spenderpackung (50) angebracht ist;
wobei die mindestens eine zweite Kammer (56) eine obere Materialschicht (52), die eine verformbare obere Wand (57) bildet, und eine an der oberen Materialschicht (52) angebrachte untere Materialschicht (54), die eine brechbare untere Wand (58) bildet, aufweist, und wobei die untere Wand Schwächemuster (59) wie Kerben, Rillen, Einschnitte, Perforationen oder eine Kombination davon aufweist, die sich nach unten bewegen sollen, um die untere Wand der mindestens einen zweiten Kammer zu brechen, wenn Druck nach unten auf die obere Wand (57) der mindestens einen zweiten Kammer (56) angewendet wird, und wobei die mindestens eine zweite Kammer (56) eine zweite Behandlungseinheit (8) enthält, um mindestens eine Substanz, die zur Behandlung einer Augenerkrankung oder eines Augenzustands verwendet wird, in das zweite Reservoir (2) abzugeben.

4. Kontaktlinsenetui nach Anspruch 3, wobei die mindestens eine erste Kammer und die mindestens eine zweite Kammer jeweils sieben Kammern für eine Wochendosis für jeweils ein Auge aufweisen.

5. Kontaktlinsenetui nach einem der Ansprüche 1 bis 4, wobei die obere Wand (57) der mindestens einen ersten Kammer (56) konvex ist.

6. Kontaktlinsenetui nach einem der Ansprüche 1 bis 5, wobei die erste Behandlungseinheit (8) eine Substanz aufweist, die zur Behandlung einer Augenerkrankung oder eines Augenzustands verwendet wird, wie aus der folgenden Gruppe ausgewählt: Augeninfektion, Augenentzündung, Erkrankung des hinteren Segments und Glaukom.

7. Kontaktlinsenetui nach einem der Ansprüche 1 bis 6, wobei die erste Behandlungseinheit (8) ein aus der folgenden Gruppe ausgewähltes nichtpharmazeutisches Augenmittel aufweist: Gleitmittel, Benetzungsmittel und Linderungsmittel, vorzugsweise Alginat oder Hyaluronsäure.

8. Set bestehend aus dem Kontaktlinsenetui nach einem der Ansprüche 1 bis 7 mit einer beliebigen Anzahl von Spenderpackungen.

9. Set nach Anspruch 8, mit einer verpackten Kontaktlinsenlösung, die dem ersten Reservoir und dem zweiten Reservoir hinzuzufügen ist.

10. Set nach einem der Ansprüche 8 oder 9, wobei die Augenerkrankung oder der Augenzustand aus der folgenden Gruppe ausgewählt ist: Augeninfektion, Augenentzündung, Erkrankung des hinteren Segments und Glaukom.

## Revendications

1. Boîte pour lentilles de contact, comprenant :
un premier conteneur (20) définissant un premier réservoir (2) ;
un premier assemblage de couvercle (30) avec un premier couvercle (40) pouvant être fixé sur le premier conteneur (20) pour fermer le premier réservoir (2), et une première batterie distributrice (50) avec au moins un premier compartiment (56) pouvant être fixé au premier couvercle (40) ou au premier conteneur (20), le premier assemblage de couvercle (30) étant prévu de telle manière qu'une paroi de sommet (57) du ou des premiers compartiments (56) soit au moins partiellement exposée sur un côté de sommet (42) du premier couvercle (40) après fixation de la première batterie distributrice (50) ;
où le ou les premiers compartiments (56) comprennent une pellicule supérieure de matériau (52) définissant une paroi de sommet (57) déformable, et une pellicule inférieure de matériau (54) fixée à la pellicule supérieure de matériau (52) définissant une paroi inférieure (58) déchirable, et où la paroi inférieure présente des motifs de moindre résistance (59) sélectionnés parmi des entailles, des marques, des découpes, des perforations ou des combinaisons de ceux-ci, prévus pour rompre en cas d'application d'une pression dirigée vers le bas sur la paroi de sommet (57) du ou des premiers compartiments (56), et où le ou les premiers compartiments (56) contiennent une première unité de traitement (8) permettant de délivrer dans le premier réservoir (2) au moins un composé utilisé pour traiter un trouble oculaire ou une affection oculaire.

2. Boîte pour lentilles de contact selon la revendication 1, où la première pellicule supérieure de matériau (52) est en matière plastique transparente ou translucide, et où la première pellicule inférieure de matériau (54) est une feuille ou est en matière plastique.

3. Boîte pour lentilles de contact selon la revendication 1 ou la revendication 2, comprenant :
un deuxième conteneur (20) définissant un deuxième réservoir (2),
un deuxième assemblage de couvercle (30) avec un deuxième couvercle (40) pouvant être fixé sur le deuxième conteneur (20) pour fermer le deuxième réservoir (2), et une deuxième batterie distributrice (50) avec au moins un deuxième compartiment (56) pouvant être fixé au deuxième couvercle (40) ou au deuxième conteneur (20), le deuxième assemblage de couvercle (30) étant prévu de telle manière qu'une paroi de sommet (57) du ou des deuxièmes compartiments (56) est au moins partiellement exposée sur un côté de sommet (42) du deuxième couvercle (40) après fixation de la deuxième batterie distributrice (50) ;
où le ou les deuxièmes compartiments (56) comprennent une pellicule supérieure de matériau (52) définissant une paroi de sommet (57) déformable, et une pellicule inférieure de matériau (54) fixée à la pellicule supérieure de matériau (52) définissant une paroi inférieure (58) déchirable, et où la paroi inférieure présente des motifs de moindre résistance (59) sélectionnés parmi des entailles, des marques, des découpes, des perforations ou des combinaisons de ceux-ci, prévus pour se déplacer vers le bas pour déchirer la paroi inférieure du ou des deuxièmes compartiments en cas d'application d'une pression dirigée vers le bas sur la paroi de sommet (57) du ou des deuxièmes compartiments (56), et où le ou les deuxièmes compartiments (56) contiennent une deuxième unité de traitement (8) permettant de délivrer dans le deuxième réservoir (2) au moins un composé utilisé pour traiter un trouble oculaire ou une affection oculaire.

4. Boîte pour lentilles de contact selon la revendication 3, où le ou les premiers compartiments et le ou les deuxièmes compartiments comprennent chacun sept compartiments pour une posologie hebdomadaire pour chaque oeil.

5. Boîte pour lentilles de contact selon l'une des revendications 1 à 4, où la paroi de sommet (57) du ou des premiers compartiments (56) est convexe.

6. Boîte pour lentilles de contact selon l'une des revendications 1 à 5, où la première unité de traitement (8) comprend un composé utilisé pour traiter un trouble oculaire ou une affection oculaire sélectionnés dans le groupe composé d'une infection oculaire, d'une inflammation oculaire, d'une lésion du fond de l'oeil et d'un glaucome.

7. Boîte pour lentilles de contact selon l'une des revendications 1 à 6, où la première unité de traitement (8) comprend un agent ophtalmique non pharmaceutique sélectionné dans le groupe composé d'agents lubrifiants, d'agents hydratants et d'agents de confort, préférentiellement, d'alginate ou d'acide hyaluronique.

8. Ensemble, comprenant :
la boîte pour lentilles de contact selon l'une des revendications 1 à 7 avec un nombre quelconque de batteries distributrices.

9. Ensemble selon la revendication 8, comprenant une solution conditionnée pour lentilles de contact à ajouter au premier réservoir et au deuxième réservoir.

10. Ensemble selon la revendication 8 ou la revendication 9, où le trouble oculaire ou l'affection oculaire sont sélectionnés dans le groupe composé d'une infection oculaire, d'une inflammation oculaire, d'une lésion du fond de l'oeil et d'un glaucome.
